# EUROPÄISCHE PATENTANMELDUNG

(11) **EP 0 994 184 A2**
(43) Veröffentlichungstag der Anmeldung: **19.04.2000**
(21) Anmeldenummer: 99117756.9
(22) Anmeldetag: 09.09.1999
(51) Int. Cl.: C12N 5/08, C12N 5/06

(54) **Kultursystem zur optimierten Anregung des Wachstums und der Differenzierung von Knorpelzellen und/oder Knochenzellen**

(30) Priorität: 18.09.1998 DE 19842802
(71) Anmelder: Gaissmaier, Christoph, Dr., 89081 Ulm (DE); Rodemann, Hans Peter, Dr., 70192 Stuttgart (DE)
(72) Erfinder: Gaissmaier, Christoph, Dr., 89081 Ulm (DE); Rodemann, Hans Peter, Dr., 70192 Stuttgart (DE)
(74) Vertreter: Herzbach, Dieter, Dipl.-Ing.

(57) **Zusammenfassung**

Die Erfindung betrifft ein Kultursystem zur optimierten Anregung des Wachstums und der Differenzierung von Knorpelzellen und/oder von Knochenzellen. Des weiteren betrifft die Erfindung ein Produkt unter Verwendung des Kultursystems sowie ein Verfahren zur Herstellung des Kultursystems.

Bei dem erfindungsgemäßen Kultursystem handelt es sich um eine Mischkultur aus einem ersten Anteil an nicht differenzierten Zellen und einem zweiten Anteil an differenzierenden und/oder differenzierten Zellen. Hierdurch wird eine notwendige zelluläre Homöostase geschaffen, die für eine gute Anregung des Wachstums von Knorpelzellen und/oder von Knochenzellen sorgt.

## Beschreibung

Die Erfindung betrifft ein Kultursystem zur optimierten Anregung des Wachstums und der Differenzierung von Knorpelzellen und/oder von Knochenzellen gemäß dem Oberbegriff des Patentanspruchs 1. Des weiteren betrifft die Erfindung ein Produkt unter Verwendung des Kultursystems, die Verwendung des Kultursystems und des Priodukts, sowie ein Verfahren zur Herstellung des Kultursystems gemäß dem Oberbegriff des Patentanspruchs 10.

Kultursysteme zur Anregung des Wachstums und der Differenzierung von Knorpelzellen und/oder von Knochenzellen sind seit vielen Jahren bekannt. Bei den bislang bekannten Kultursystemen werden die Zellen zur Anregung des Wachstums von Knorpelzellen und/oder von Knochenzellen in Monokulturen verwendet, d. h. es finden nur Zellen eines einheitlichen Differenzierungsgrades Verwendung. Den bislang bekannten Kultursystemen werden zur Optimierung der zellulären Bildung von Knorpelzellen und/oder Knochenzellen Wachstumsfaktoren, sogenannte growth factors, zugesetzt.

Die bislang bekannten Kultursysteme gewährleisten jedoch nur eine unzureichende Anregung des Wachstums und der Differenzierung von Knorpelzellen und/oder von Knochenzellen, da die für die Bildung von Knorpelzellen und/oder von Knochenzellen erforderlichen Wachstumsfaktoren noch nicht hinreichend erforscht sind. Desweitern verfügt die exogene Zugabe der Wachstumsfaktoren über den Nachteil, daß die verwendeten Wachstumsfaktoren über unterschiedliche biologische Halbwertszeiten verfügen und daher eine zeitlich konstante Anregung nicht gewährleistet ist.

Der vorliegenden Erfindung liegt daher das Problem zu Grunde, ein Kultursystem bereitzustellen, welches eine optimierte Bildung von Knorpelzellen und/oder von Knochenzellen gewährleistet.

Zur Lösung dieses Problems verfügt das erfindungsgemäße Kultursystem über die Merkmale des Patentanspruchs 1. Durch die Verwendung einer Mischkultur aus einem ersten Anteil an nicht differenzierten Zellen und einem zweiten Anteil an differenzierenden und/oder differenzierten Zellen wird eine notwendige zelluläre Homöostase geschaffen, die für eine gute Anregung des Wachstums und der Differenzierung von Knorpelzellen und/oder von Knochenzellen sorgt.

Bei den differenzierenden und/oder differenzierten Zellen des zweiten Anteils handelt es sich um Zellen, die zur Differenzierung speziell stimuliertet wurden. Hierauf wird weiter unten noch in größerem Detail eingegangen.

Vorzugsweise handelt es sich bei den Zellen des ersten Anteils der Mischkultur um proliferative, potentiell fibroblastoide, chondrogene und/oder osteogene Zellen und bei den Zellen des zweiten Anteils um nicht-proliferative, fibroblastoide, chondrogene und/oder osteogene Zellen. Die Verwendung von Zellen unterschiedlichen Differenzierungs- und Funktionszustands gewährleistet eine Optimierung des erfindungsgemäßen Kultursystems (es handelt sich hierbei um ein in vitro System) gemäß einer in vivo Siutuation.

Das erfindungsgemäße Produkt unter Verwendung des Kultursystems verfügt über die Merkmale des Patentanspruchs 7 und das erfindungsgemäße Verfahren zur Herstellung des Kultursystems des verfügt über die Merkmale Patentanspruchs 10.

Weitere bevorzugte Ausgestaltungen der Erfindung ergeben sich aus den Unteransprüchen und der nachfolgenden Beschreibung. An Hand der Zeichnung wird ein Ausführungsbeispiel der Erfindung beschrieben. In der Zeichnung zeigt:

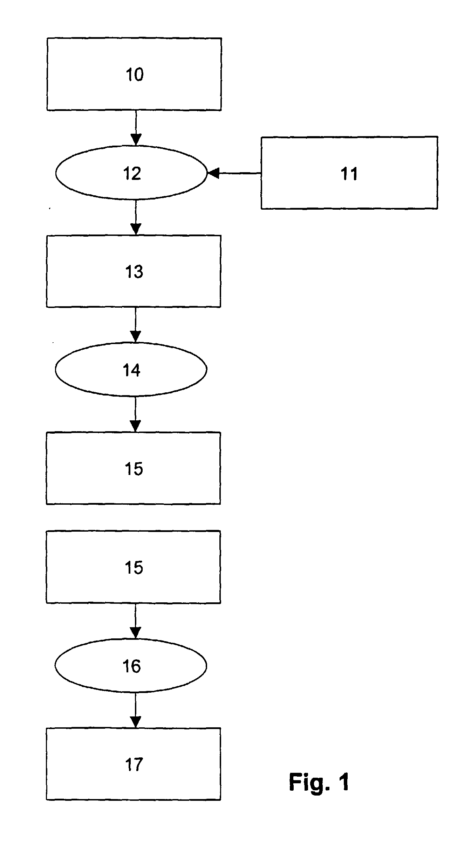

- Fig.1:: ein schematisiertes Blockdiagramm zur Verdeutlichung des Herstellungsverfahrens für das erfindungsgemäße Kultursystem.

Bei dem erfindungsgemäßen Kultursystem zur Anregung des Wachstums und der Differenzierung von Knorpelzellen und/oder von Knochenzellen handelt es sich um eine Mischkultur. Die Mischkultur besteht aus Zellen unterschiedlichen Differenzierungs- und Funktionszustands. Bei einem ersten Anteil von Zellen der Mischkultur handelt es sich um nicht differenzierte, proliferative, potentiell fibroblastoide, chondrogene und/oder osteogene Zellen. Bei einem zweiten Anteil von Zellen der Mischkultur handelt es sich um differenzierende und/oder differenzierte, nicht-proliferative, fibroblastoide, chondrogene und/oder osteogene Zellen. Die Zellen des zweiten Anteils wurden zur Differenzierung speziell stimuliertet. Durch diese erfindungsgemäße Mischkultur wird die notwendige zelluläre Homöostase geschaffen, wobei eine Optimierung des erfindungsgemäßen Kultursystems gemäß einer in vivo Siutuation gewährleistet ist. Bei dem erfindungsgemäßen Kultursystem handelt es sich um ein in vitro System.

Das erfindungsgemäße Kultursystem bzw. die Mischkultur verfügt vorzugsweise über Zellen nach folgender Zusammensetzung:
40 % bis 99 % bzgl. des ersten Anteils an Zellen,
1 % bis 60 % bzgl. des zweiten Anteils an Zellen.

Bevorzugt ist folgende Zusammensetzung der Mischkultur:
90 % bzgl. des ersten Anteils an Zellen,
10 % bzgl. des zweiten Anteils an Zellen.

Die erfindungsgemäße Mischkultur kann in zweidimensionalen Systemen, z. B. einer Nährlösung, oder dreidimensionalen Systemen Verwendung finden. Liegt die obige, bevorzugte Mischkultur in einer Nährlösung vor, so handelt es sich demzufolge bei 90 % der Zellen der Mischkultur um nicht differenzierte, proliferative, potentiell fibroblastoide, chondrogene und/oder osteogene Zellen und bei 10 % der Zellen der Mischkultur um differenzierende und/oder differenzierte, nicht-proliferative, fibroblastoide, chondrogene und/oder osteogene Zellen.

Besonders vorteilhaft ist ein Produkt aus der obigen Mischkultur und einem dreidimensionalen Träger für die Mischkultur. Der Träger kann aus einem resorbierbaren Kunststoff, insbesondere aus Polylactid oder Polyglykol, gebildet sein. Alternativ kann der Träger aus einem resorbierbaren Biopolymer, insbesondere aus Kollagen oder Hyaluronsäurederivat, gebildet sein.

Das Verfahren zur Herstellung / Gewinnung des erfindungsgemäßen Kultursystems bzw. der Mischkultur wird nachfolgend im Detail unter Bezugnahme auf Fig. 1 beschrieben:

Als Ausgangssubstanz 10 zur Herstellung der Mischkultur dient Gewebe mesenchymalen Ursprungs. Bei diesem Gewebe mesenchymalen Ursprungs handelt es sich vorzugsweise um mittels Biopsie-Entnahme gewonnenes Knorpelgewebe und/oder Knochengewebe. Es kann sich jedoch auch um anderes weichteiliges Bindegewebe handeln. Ferner kann stromales Knochenmark als Ausgangssubstanz 10 verwendet werden.

Diese Ausgangssubstanz 10 wird mit einer Nährlösung 11 zusammengeführt, wobei es sich bei der Nährlösung 11 um eine gepufferte, gewebedissoziierende Enzyme enthaltende Nährlösung handelt. Das Zusammenführen der Ausgangssubstanz 10 und der Nährlösung 11 ist in Fig. 1 durch einen Verfahrensschritt 12 dargestellt. Bei dem Verfahrensschritt 12 handelt es sich sozusagen um einen Mischschritt. Als Ergebnis des Verfahrensschritts 12 liegt ein Zwischenprodukt 13 vor, bei dem es sich demnach um die in der Nährlösung 11 befindliche Ausgangssubstanz 10 handelt.

Bei der Verwendung von z. B. Knorpelgewebe und/oder Knochengewebe als Ausgangssubstanz 10 umfaßt die Ausgangssubstanz 10 nicht differenzierte, proliferative Zellen und differenzierte, nicht-proliferative Zellen. Um diese Zellen aus dem Gewebeverband zu lösen, wird die Ausgangssubstanz 10 mit der Nährlösung 11 zusammengeführt. Die aus diesem Verfahrensschritt 12 erhaltene Zellsuspension (Zwischenprodukt 13) enthält alle aus dem Gewebe isolierten nicht differenzierten, proliferativen Zellen und differenzierten, nicht-proliferativen Zellen.

Auf die Zellsuspension wird ein weiterer Verfahrensschritt 14 angewendet um die nicht differenzierten, proliferativen Zellen zu gewinnen. Als Verfahrensschritt 14 kann jede dem hier angesprochenen Fachmann bekannte Vermehrungsmethode eingesetzt werden. Hierbei vermehren sich die nicht differenzierten Zellen während sich die differenzierten Zellen nicht vermehren. Mit anderen Worten werden also die ursprünglich vorhandenen differenzierten, nicht-proliferativen Zellen derart verdünnt, daß nach vollendetem Verfahrensschritt 14 im wesentlichen nur noch nicht differenzierte Zellen vorliegen. Hierbei handelt es sich dann um das in Fig. 1 dargestellte erste Produkt 15.

An dieser Stelle sei bereits angemerkt, daß die durch die so gewonnenen, nicht differenzierten Zellen in der Mischkultur die Zellen des ersten Anteils an Zellen bilden.

Zur Gewinnung der differenzierenden und/oder differenzierten Zellen für den zweiten Anteil an Zellen der erfindungsgemäßen Mischkultur wird ein Teil des ersten Produkts 15, also der nicht differenzierten Zellen, einem weiteren Verfahrensschritt 16 unterzogen. Bei diesem Verfahrensschritt 16 handelt es sich um einen Stimulierungsschritt, mit Hilfe dessen die nicht differenzierten Zellen zur Differenzierung stimuliert werden. Als Stimulierungsschritt kommt beispielsweise ein zeitlich definierter Serumentzug in Frage. Auch kann die Stimulierung durch eine Behandlung mit Cytokinen, insbesondere mit Cytokinen der TGF-β-Superfamilie, erfolgen. Des weiteren kann die Stimulierung durch elektromagnetische Befeldung durchgeführt werden. Auch kommt eine Behandlung mit chemischen und physikalischen Agenzien, wie z. B. mit Cytostatika, UV-Strahlung oder ionisierender Strahlung, in Betracht. Die Details der als Stimulierungsschritt in Betracht kommenden Methoden sind dem hier angesprochenen Fachmann geläufig und bedürfen daher keiner weiteren Erläuterung.

Als Ergebnis des Verfahrensschritts 16, also des Stimulierungsschritts, gewinnt man aus dem ersten Produkt 15, den nicht differenzierten Zellen, ein zweites Produkt 17, wobei es sich bei dem zweiten Produkt 17 um die differenzierenden und/oder differenzierten Zellen bzw. um die zur Differenzierung stimulierten Zellen handelt, die sich ebenfalls in Nährlösung befinden.

Die durch den Stimulierungsschritt gewonnenen differenzierenden und/oder differenzierten Zellen bilden in der Mischkultur die Zellen des zweiten Anteils an Zellen.

Zur Herstellung des erfindungsgemäßen Kultursystems werden die Produkte 15 und 17, also die in Nährlösung befindlichen nicht differenzierten Zellen und die zur Differenzierung stimulierten, differenzierenden und/oder differenzierten Zellen gemäß dem gewünschten Zusammensetzungsverhältnis des Kultursystems gemischt.

Soll das erfindungsgemäße Kultursystems in Verbindung mit einem dreidimensionalen Träger aus z. B. resorbierbarem Kunststoff Verwendung finden, so kann die nach dem obigen Verfahren gewonnene Mischkultur auf den dreidimensionalen Träger aufgebracht werden, z. B. durch Beträufeln oder dergleichen.

## Patentansprüche

1. Kultursystem zur optimierten Anregung des Wachstums und der Differenzierung von Knorpelze!len und/oder von Knochenzellen, **gekennzeichnet durch** einen ersten Anteil an nicht differenzierten Zellen und einen zweiten Anteil an differenzierenden und/oder differenzierten Zellen.

2. Kultursystem nach Anspruch 1, **dadurch gekennzeichnet, daß** es sich bei den differenzierenden und/oder differenzierten Zellen des zweiten Anteils um zur Differenzierung stimulierte Zellen handelt.

3. Kultursystem nach Anspruch 1 oder 2, **dadurch gekennzeichnet, daß** die Zellen des ersten Anteils und des zweiten Anteils chondrogene und/oder osteogene Zellen sind.

4. Kultursystem nach einem oder mehreren der Ansprüche 1 bis 3, **dadurch gekennzeichnet, daß** die Zellen des ersten Anteils proliferative, potentiell fibroblastoide Zellen sind, und daß die Zellen des zweiten Anteils nicht-proliferative, fibroblastoide Zellen sind.

5. Kultursystem nach einem oder mehreren der Ansprüche 1 bis 4, **dadurch gekennzeichnet, daß** der erste Anteil an Zellen zwischen 40 % und 99 % an den Zellen des Kultursystems beträgt, und daß der zweiten Anteil an Zellen zwischen 1 % und 60 % an den Zellen des Kultursystems beträgt.

6. Verwendung des Kultursystems nach einem oder mehreren der Ansprüche 1 bis 5 zur Anregung des Wachstums und der Differenzierung von Knorpelzellen und/oder von Knochenzellen.

7. Produkt aus einem Kultursystem nach einem oder mehreren der Ansprüche 1 bis 5 und einem dreidimensionalen Träger für das Kultursystem.

8. Produkt nach Anspruch 7, **dadurch gekennzeichnet, daß** der Träger aus einem resorbierbaren Kunststoff, insbesondere aus Polylactid oder Polyglykol, oder aus einem resorbierbaren Biopolymer, insbesondere aus Kollagen oder Hyaluronsäurederivat, gebildet ist.

9. Verwendung des Produkts nach Anspruch 7 oder 8 zur Anregung des Wachstums und der Differenzierung von Knorpelzellen und/oder von Knochenzellen.

10. Verfahren zur Herstellung eines Kultursystems, das der Anregung des Wachstums von Knorpelzellen und/oder von Knochenzellen dient, **dadurch gekennzeichnet, daß:**
a) aus einer Ausgangssubstanz mit Hilfe einer Isolationsmethode nicht differenzierte Zellen gewonnenen werden,
b) nicht differenzierte, mit Hilfe des Schritts a) gewonnene Zellen einem Stimulierungsschritts unterzogen werden, wobei hiermit differenzierende und/oder differenzierte Zellen gewonnen werden,
c) die mit Hilfe des Schritts a) gewonnenen, nicht differenzierten Zellen und die mit Hilfe des Schritts b) gewonnenen, differenzierenden und/oder differenzierten Zellen nach einem vorbestimmten Mischungsverhältnis gemischt werden.

11. Verfahren nach Anspruch 10, **dadurch gekennzeichnet, daß** die Ausgangssubstanz vor der Behandlung mit der Isolationsmethode mit einem Nährmedium gemischt wird.
